# NEW EUROPEAN PATENT SPECIFICATION

(11) **EP 0 180 309 B2**
(45) Date of publication and mention of the opposition decision: **17.12.1997**
(45) Mention of the grant of the patent: 19.02.1992
(21) Application number: 85306391.5
(22) Date of filing: 09.09.1985
(51) Int. Cl.: A01N 47/44, A61L 2/18, G02C 13/00

(54) **Improved disinfecting and preserving solutions for contact lenses and methods of use**
Desinfektions- und Aufbewahrungslösungen für Kontaktlinsen und Verfahren zur Verwendung
Solutions pour la désinfection et la conservation des lentilles de contact et procédés d'utilisation

(30) Priority: 28.09.1984 US 655965; 11.12.1984 US 680450
(43) Date of publication of application: 07.05.1986
(73) Proprietor: BAUSCH & LOMB INCORPORATED, Rochester, New York 14604-2701 (US)
(72) Inventor: Ogunbiyi, Lai, Fairport, NY 14450 (US); Smith, Francis X., Walworth, NY 14563 (US); Riedhammer, Thomas M., Toms River, NJ 08753 (US)
(74) Representative: Allam, Peter Clerk

(56) References cited:
- EP-A- 0 076 136
- FR-A- 2 182 962
- FR-A- 2 517 208
- US-A- 4 031 576
- US-A- 4 354 952
- P. LUMBROSO et al., "Dictionnaire des produits d'entretien en contactologie", 1st edition, 9th April 1982, La Source d'Or, Marsat, FR.
- M.J. Sibley and O.H. Dabezies Jr., "Soft Lens Hygiene", in Contact Lenses: the CLAO Guide to Basic Science and Clinical Practice, Grune & Stratton, 1984, pages 40.1 to 40.17
- S.S. Block, Disinfection, Sterilization and Preservation 3rd ed., pages 335-45. Lea & Febiger Philadelphia 1983

## Description

This invention relates to disinfecting and preserving solutions for the treatment of contact lenses and for other purposes and to methods of use.

Generally, contact lenses in wide use fall into two categories: the hard or rigid corneal type lenses formed from materials prepared by polymerization of acrylic esters, such as polymethyl methacrylate (PMMA), and gel, hydrogel or soft type lenses made of polymerized hydrophilic or hydrophobic monomers, such as 2-hydroxyethyl methacrylate (HEMA). The hard acrylic type contact lenses are characterized by low water vapor diffusion constants, resistance to the affects of light, oxygen and hydrolysis and absorb only minor amounts of aqueous fluids. Because of the durability of hard contact lenses, coupled with their tendency not to absorb appreciable amounts of water, the selection of suitable disinfecting agents, cleaning agents or other lens care compounds is relatively non-critical.

However, unlike hard lenses, sort type contact lenses and certain of the newer gas permeable hard contact lenses have a tendency to bind and concentrate significantly more fluids, environmental pollutants, water impurities, as well as antimicrobial agents and other active ingredients commonly found in lens care solutions. In most instances, the low levels of the ingredients in lens care solutions do not lead to eye tissue irritation when used properly. Nevertheless, because of the inherent binding action of protein deposits and sort lens materials disinfecting agents and preservatives tend to build up on lens surfaces and become concentrated to potentially hazardous levels, such that when released can cause corneal inflammation and other eye tissue irritation.

Previous efforts to alleviate the problem of binding and concentrating disinfectants and preservatives onto contact lens surfaces, and reducing the potential for eye tissue irritation have not been totally satisfactory. For example, in spite of low toxicity levels not all disinfectants are compatible for use with all types of contact lenses. Many hard lens disinfecting and preservative solutions contain benzalkonium chloride or chlorobutanol. Although they are effective antibacterial agents, their use can result in a loss of lens hydrophilic properties, cause solution instability or may even lack compatibility with certain types of hard lenses, e.g. high silicon content.

Other antibacterial agents were found to be more compatible with contact lenses and exhibit less binding on lens surfaces. In one case, it was found that chlorhexidine, a biguanide, binds to soft lens material seven times less than benzalkonium chloride, but the presence of proteinaceous oily tear-film deposits can double the amount of chlorhexidine absorbed over that of clean lenses. U.S. patent 4,354,952 discloses very dilute disinfecting and cleaning solutions containing chlorhexidine or its salts in combination with certain amphoteric and non-ionic surfactants. These solutions were found to reduce the amount of binding of chlorhexidine on hydrophilic sort contact lenses. Notwithstanding the reduction in binding achieved by this invention, the use of chlorhexidine did result in certain tradeoffs. That is, the antimicrobial activity of the chlorhexidine may be diminished when used with certain amphoteric surfactants. Furthermore, if not used in proper ratio, the surfactant and disinfectant will precipitate unless a non-ionic type surfactant is also employed.

U.S. patent 4,361,548 discloses a contact lens disinfectant and preservative containing dilute aqueous solutions of a polymer; namely, dimethyldiallylammonium chloride (DMDAAC) having molecular weights ranging from about 10,000 to 1,000,000. Amounts of DMDAAC homopolymer as low as 0.00001 percent by weight may be employed when an enhancer, such as thimerosal, sorbic acid or phenylmercuric salt is used therewith. Although lens binding and concomitant eye tissue irritation with DMDAAC were reduced, it was found in some users to be above desirable clinical levels.

British patent 1,432,345 discloses contact lens disinfecting compositions containing a polymeric biguanide (of the type contemplated by the present invention and a mixed phosphate buffer. The products embraced by this patent have not found acceptance by the consumer. Corneal staining is an indication of patient acceptability and compositions as disclosed by this patent have staining values of 17% or more present, far above that which is desirable for patient acceptability, see Table V in the Examples of this application.

Other efforts to reduce or eliminate sort lens binding have led to the use of anti-binding or detoxifying agents, like polyvinyl pyrrolidone (PVP) and polyvinyl alcohol (PVA). However, these polymers alone were found to be ineffective, for the most part, in reducing lens binding and eye tissue irritation.

Accordingly, there is a need for improved disinfecting and preservative solutions which are compatible for use with most types of contact lenses while maintaining both a high level of antibacterial activity and low order of toxicity to eye tissue with little or no binding or concentrating of the disinfecting agent onto lens surfaces.

The present invention provides improved solutions for disinfecting and/or preserving contact lenses. The solutions are compatible with both hard and sort type lenses, and are adaptable for use with virtually any of the commonly known disinfecting techniques, including "cold" soaking under ambient temperature conditions, as well as with high temperature disinfecting methods. The disinfecting and preservative solutions of the present invention are especially noteworthy for their wide spectrum of bactericidal and fungicidal activity at low concentrations coupled with very low toxicity and reduced affinity for binding and concentrating when used with soft type contact lenses.

More particularly, in accordance with this invention there is provided an aqueous solution for disinfecting and/or preserving contact lenses comprising (a) a microbicidally effective amount, within the range from 0.000001 to 0.0003 weight percent, of a biguanide, or a water-soluble salt thereof, having the following general formula (I): 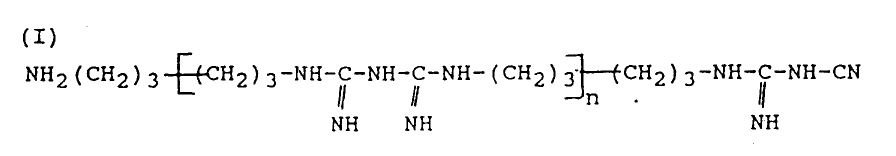 wherein n is from 1 to 500, and (b) a buffer, characterized in that said buffer is a borate buffer.

The present invention also provides a method of disinfecting and/or preserving contact lenses, which comprises contacting the lenses with a germicidally effective amount of the solution defined in the preceding paragraph.

The biguanides of formula (I) for use in the present invention are hexamethylene biguanides, their polymers and water-soluble salts of such base compounds and polymers. Generally, the polymers have molecular weights of up to about 100,000. Typically, the solutions will be made isotonic with lacrimal fluids. The antibacterial action of the biguanide-containing solutions described herein may also be supplemented by the addition of other germicidal agents. Because the overall germicidal activity of such combinations will in some instances be greater than when each is used separately, the concentration of total disinfectant in solution can be lowered, further reducing the potential for binding, concentrating and adverse toxic reactions.

The disinfecting solutions of the present invention are effective at low concentrations against a wide spectrum of microorganisms, including hut not limited to S. epidermidis, C. Albicans, A. fumigatus, etc.

More particularly, the present invention includes the use of low molecular weight oligomers where n averages from 2 to 7, high molecular weight long chain polymers up to approximately 100,000 M.W., as well as the individual monomer of such oligomers and polymers ie where n is 1. In addition to the above, the present invention also includes the use of water-soluble salts of the free bases, such as hydrochloride and borate salts, acetate, gluconate, sulfonate, tartrate and citrate salts. One preferred group of water soluble biguanides will have average molecular weights of at least 1,000 and more preferably will have average molecular weights from 1,000 to 50,000.

The range of polymeric biguanides within the foregoing broad definition for use in the solutions of the present invention is rather surprising and unexpected, since polymers in the higher molecular weight ranges usually demonstrate less binding and lower toxicity levels than corresponding lower molecular weight materials.

The above-disclosed biguanides and methods of preparation are described in the literature. For example. U.S. patent 3,428,576 describes the preparation of such biguanides from a diamine and salts thereof and a diamine salt of dicyanimide. This patent expressly teaches methods for making, e.g. the hydrochloride salt of polyhexamethylene biguanide which is also commercially available from ICI Americas. Inc. under the trademark Cosmocil CQ. For convenience purposes only, the biguanides described hereinafter for disinfecting and/or preserving contact lenses shall be referred to as "PHMB".

The preserving solutions of the instant invention are effective in concentrations as low as 0.000001 weight percent PHMB. Generally, the disinfectant and preservative solutions will contain from about 0.00001 to about 0.0003 weight percent (i.e. three parts per million -ppm) PHMB. It has also been found that the bactericidal activity of the solutions may be enhanced or spectrum of activity broadened through the use of a potentiating amount of a second disinfectant or germicidal agent. As a result, the total concentration of disinfectant required when PHMB is used on combination with other germicidal agents may be lowered further due to complimentary bactericical activity, which is most desirable in achieving the lowest possible potential for lens binding, concentrating and eye tissue inflammation. Thus, the effective concentration of PHMB may be as low as about 0.000001 weight percent and up to about 0.0003 weight percent.

The disinfecting/preserving solutions of this invention contain a borate buffer, e.g. boric acid, sodium borate, potassium tetraborate, potassium metaborate or mixtures of the same. Applicants were surprised to find that, at the amounts of PHMB present in the solutions of this invention, a borate buffer system was exceptionally effective. This is particularly surprising in view of British Patent 1,432,345 disclosing mixed phosphate buffers for solutions containing higher amounts of PHMB. Whatever the reasons, PHMB solutions buffered only with phosphate and containing no amphoteric surfactant are ineffective at very low concentrations. See Table I of the Examples below.

Generally, the borate buffers may be used in amounts ranging from about 0.05 to 2.5 percent, and more preferably, from about 0.1 to 1.5 percent (w/w).

The solutions of this invention may additionally include a sequestering agent, a non-ionic surfactant and/or a cationic surfactant, to act as a buffer enhancer.

Suitable sequestering agents include ethylene diametetraacetic acid, gluconic acid, citric acid, tartaric acid and their salts, e.g. sodium salts.

The foregoing non-ionic and cationic surfactants, when employed as a buffer enhancer, will be present in an amount from 0.0001 percent to 5.0 percent (w/w).

Additionally, the non-ionic surfactant can be employed both as a buffer enhancer and a cleaning agent in a combined cleaning and disinfecting/preserving solution.

As suitable cationic surfactants may be mentioned dual quaternary ammonium compositions are described in U.S. patents 3,525,793 and 3,472,939 and are commercially available from Onyx Chemical Company, Jersey City, New Jersey under the trade mark BTC 2125M.

A second disinfectant/germicide can be employed as a solution preservative, but it may also function to potentiate, complement or broaden the spectrum of microbicidal activity of the PHMB. This includes microbicidally effective amounts of germicides which are compatible with and do not precipitate in the presence of PHMB, and comprises concentrations ranging from about 0.00001 to about 0.5 weight percent. and more preferably, from about 0.0001 to about 0.1 weight percent. Suitable complementary germicidal agents include, but are not limited to thimerosal, sorbic acid, 1,5-pentanedial, alkyl triethanolamines, phenylmercuric salts, e.g. nitrate, borate, acetate, chloride and mixtures thereof. Other germicidal compounds and salts may be used. Suitable salts are soluble in water at ambient temperature to the extent of at least 0.5 weight percent. These salts include the gluconate, isothionate, (2-hydroxyethanesulfonate), formate, acetate, glutamate, succinanate, monodiglycollate, dimethanesulfonate, tactate, diisobutyrate and glucoheptonate.

Further embodiments of potentiating or complementary disinfecting agents for use with PHMB also include certain quaternary ammonium compounds which possess a generally wide spectrum of bactericidal activity and wetting properties. Representative examples of the quaternary ammonium compounds are compositions comprised of balanced mixtures of n-alkyl dimethyl benzyl ammonium chlorides.

Generally, the aqueous solutions of the present invention for treating contact lenses are also adjusted with tonicity agents to approximate the osmotic pressure of normal lacrimal fluids which is equivalent to a 0.9 percent solution of sodium chloride or 2.5 percent of glycerol solution. The solutions are made substantially isotonic with physiological saline used alone or in combination, otherwise if simply blended with sterile water and made hypotonic or made hypertonic the lenses will lose their desirable optical parameters. Correspondingly, excess saline may result in the formation of a hypertonic solution which will cause stinging and eye irritation.

The aqueous isotonic solutions of PHMB with optional germicidal agents are useful disinfectants for both hard and soft contact lenses without any further additives. Nevertheless, the solutions of the present invention may be formulated into specific contact lens care products, such as wetting solutions, soaking solutions, cleaning and conditioning solutions, as well as all purpose type lens care solutions, etc. and mixtures thereof. Such additives make the solutions more acceptable to the user in terms of greater comfort. However, the additives must be non-toxic and compatible with contact lenses.

When used, non-ionic surfactants impart cleaning and conditioning properties and are usually present in amounts up to 15 weight percent. The surfactant should be soluble in the lens care solution, non-irritating to eye tissues and still usually have a hydrophilic-lipophile balance (HLB) of 12.4 to 18.8. Satisfactory non-ionid surfactants include polyethylene glycol esters of fatty acids, e.g. coconut, polysorbate, polyoxyethylene or polyoxypropylene ethers of higher alkanes (C₁₂-C₁₈). Examples of the preferred class include polysorbate 20 (available under the trademark Tween 20), polyoxyethylene (23) lauryl ether (Brij® 35), polyoxyethylene (40) stearate (Myrj® 52), polyoxyethylene (25) propylene glycol stearate (Atlas® G 2612).

One non-ionic surfactant in particular, consisting of a poly(oxypropylene)-poly(oxyethylene) adduct of ethylene diamine having a molecular weight from about 7,500 to about 27,000 wherein at least 40 weight percent of said adduct is poly(oxyethylene), has been found to be particularly advantageous for use in cleaning and conditioning both soft and hard contact lenses when used in amounts from about 0.01 to about 15 weight percent. The CTFA Cosmetic Ingredient Dictionary's adopted name for this group of surfactants is poloxamine. Such surfactants are available from BASF Wyandotte Corp., Wyandotte, Michigan, under the registered trademark "Tetronic". An analoguous of series of surfactants is the poloxamer series which is a poly(oxyethylene), poly(oxypropylene) block polymers available under the trademark "Pluronic".

Other amphoteric, cationic and nonionic surfactants suitable for in the invention can be readily ascertained, in view of the foregoing description, from McCutcheon's Detergents and Emulsifiers, North American Edition, McCutcheon Division, MC Publishing Co., Glen Rock, NJ 07452.

It may also be desirable to include water-soluble viscosity builders in the PHMB-containing solutions of the present invention. Because of their demulcent effect, viscosity builders have a tendency to enhance the lens wearer's comfort by means of a film on the lens surface cushioning impact against the eye. Included among the water-soluble viscosity builders are the cellulose polymers like hydroxyethyl or hydroxypropyl cellulose, carboxymethyl cellulose and the like. Such viscosity builders may be employed in amounts ranging from about 0.01 to about 4.0 weight percent or less.

This invention relates to disinfecting and/or preserving solutions for use with most contact lenses, including hard and sort lenses, as well as the newer hard gas permeable type contact lenses, such as described in U.S. patent 4,327,203. The term "soft contact lens" as used herein generally refers to those contact lenses which readily flex under small amounts of force and return to their original shape when that force is released. Typically, soft contact lenses are formulated from poly(hydroxyethyl methacrylate) which has been, in the preferred formulations, crosslinked with ethylene glycol dimethacrylate. For convenience, this polymer is generally known as PHEMA. Soft contact lenses are also made from silicon polymers crosslinked, for example, with dimethyl polysiloxane. Conventional "hard contact lenses", which cover only the cornea of the eye, usually consist of poly(methyl methacrylate) crosslinked with ethylene glycol dimethacrylate.

The aqueous PHMB-containing solutions can be effectively used in disinfecting contact lenses by any of the well recognized methods. For example, lenses may be treated by the "cold" soaking method at room temperature for a period ranging from 4 to 12 hours. The lenses are then removed from the solution, washed in preserved isotonic saline solution and then replaced on the eye.

In addition to the cold soaking method, the solutions disclosed herein are adaptable for use in other types of equipment, such as ultrasonic cleaners. Because the solutions are also stable when heated, they are adaptable for use with high temperature disinfecting methods. Typically, lenses are heated to 80° in a disinfecting unit containing the solution for a time period of at least 10 minutes, removed and rinsed with isotonic saline.

The present invention includes the use of the PHMD and buffer preservative combination in ophthalmologic products and in dermatologic formulations applied near the eye. Such use will, of course, depend upon the compatibility of the preservative combination with the active ingredient(s) in the product.

The following Examples illustrate the compositions and methods of the instant invention.

### EXAMPLE I

An aqueous contact lens disinfectant solution is prepared having the following formulation:

| | Percent (w/v) |
|---|---|
| Polhexamethylene Biguanide HCl* | .0001 |
| Poloxamine 1107** | .5 |
| Na₂EDTA | .011 |
| Boric Acid | 1.10 |
| Sodium Borate | .40 |
| Sodium Chloride | .30 |
| Distilled Water qs | 100.0 |

| | |
|---|---|
| * n = 4.5 to 6.5 | |
| ** Flake grade, molecular weight 14,500, 70% (wt.) poly(oxyethylene) Tetronic® 1107, BASF Wyandotte Corp. | |

The solution is prepared by gradually heating approximately 80 percent of the water to 80°C while dissolving the disodium EDTA therein. The boric acid and sodium borate are added to the heated solution of disodium EDTA and dissolved. The sodium chloride is then added to the solution and dissolved, followed by the addition of surfactants. After the solution is cooled to room temperature, the polyhexamethylene biguanide is added, followed by the balance of distilled water. The solution is sterilized by forcing through an 0.22 micron cellulose acetate filter by means of a peristaltic pump and packaged in sterilized plastic containers.

The bactericidal activity of the above solution is tested by exposing S. epidermidis (1.6x10⁶ microorganisms/ml) and C. albicans (1.1x10⁶ microorganisms/ml) each to 20 ml of said solution at room temperature for 5 hours. Subsequently, an aliquot sample of each is placed on an agar plate and incubated for 48 hours at elevated temperatures. At the conclusion of the incubation period, the plates are examined for the development of colonies. The results showed 6 log reduction of S. epidermidis microorganisms and 1.3 log reduction of C. albicans microorganisms.

An aqueous contact lens disinfecting solution is prepared with the following formulation:

### EXAMPLE II

| | Percent (w/v) |
|---|---|
| Polyhexamethylene Biguanide HCl* | .0001 |
| Na₂ EDTA | .11 |
| Boric Acid | 1.1 |
| Sodium Borate | .40 |
| Sodium Chloride | .30 |
| Distilled Water qs | 100.0 |

| | |
|---|---|
| * n = 4.5 to 5.6 | |

The above formulation may be prepared by the method described in Example I.

### EXAMPLE III

An aqueous contact lens disinfecting solution is prepared with the following formulation:

| | Percent (w/v) |
|---|---|
| Polyhexamethylene Biguanide HCl* | .00009 |
| Poloxamine 1107 | .5 |
| Na₂ EDTA | .11 |
| Boric Acid | .64 |
| Sodium Borate | .16 |
| Sodium Chloride | .49 |
| Distilled Water qs | 100.0 |

| | |
|---|---|
| * n = 500 | |

The above formulation may be prepared by the method described in Example I.

### EXAMPLE IV

An aqueous contact lens disinfecting solution is prepared with the following formulation:

| | Percent (w/v) |
|---|---|
| Polyhexamethylene Biguanide HCl* | .00009 |
| Na₂ EDTA | .11 |
| Boric Acid | .64 |
| Sodium Borate | .16 |
| Sodium Chloride | .49 |
| Distilled Water qs | 100.0 |

| | |
|---|---|
| * n = 50 | |

The solution is prepared by dissolving the sodium borate in approximately 80 percent of the distilled water. The disodium EDTA is then added to the sodium borate solution, followed by dissolving the boric acid and sodium chloride therein. The polyhexamethylene biguanide is added, followed by the balance of distilled water. The solution may be sterilized according to the method described in Example I.

### EXAMPLE V

In this example, the effectiveness of polyhexamethylene biguanide hydrochloride (n = 4.5 to 6.5) as a preserving agent is evaluated, using (1) a borate buffer system and (2) a mixed phosphate buffer system. Each preserved solution is prepared by the method of Example I and each is adjusted so as to be isotonic. All ingredients are by weight percent unless otherwise noted. Following the microbial test methods of Example I, each solution is evaluated for preservative effectiveness against S. aureus, P. aeruginosa and E. coli organisms after 14 and after 28 days. To be considered effective in this test, there must be at least 3 log reduction in number of organisms for each type of organism at 14 days and at 28 days. The solutions and test results are tabulated in Table I (Solutions) and Table IA (Results) below.

**TABLE I**

| PHMB PRESERVED SOLUTIONS | | | | | | | |
|---|---|---|---|---|---|---|---|
| Sol.# | FORMULATION⁽¹⁾ | | | | | | |
| | Boric Acid | Sodium Borate | Sodium Chloride | Na₂ EDTA | (PPM) PHMB | Dibasic Phosphate | Monobasic Phosphate |
| 1 | 1.1% | 0.40% | 0.30% | 0.11% | 0.99 | -- | -- |
| 2 | 1.1% | 0.40% | 0.30% | 0.11% | 0.88 | -- | -- |
| 3 | 1.1% | 0.40% | 0.30% | 0.11% | 0.77 | -- | -- |
| 4 | 1.1% | 0.40% | 0.30% | 0.11% | 0.66 | -- | -- |
| 5 | 1.1% | 0.40% | 0.30% | 0.11% | 0.55 | -- | -- |
| 6 | 1.1% | 0.40% | 0.30% | 0.11% | 0.44 | -- | -- |
| 7 | 1.1% | 0. 40% | 0.30% | 0.11% | 0.33 | -- | -- |
| 8 | 1.1% | 0.40% | 0.30% | 0.11% | 0.22 | -- | -- |
| 9 | 1.1% | 0.40% | 0.30% | 0.11% | 0.11 | -- | -- |
| 10 | 1.1% | 0.40% | 0.30% | 0.11% | 0.05 | -- | -- |
| *11 | -- | -- | 0.55%. | 0.11% | 0.99 | 0.65% | 0.1% |
| *12 | -- | -- | 0.55% | 0.11% | 0.88 | 0.65% | 0.1% |
| *13 | -- | -- | 0. 55% | 0.11% | 0.77 | 0.65% | 0.1% |
| *14 | -- | -- | 0.55% | 0.11% | 0.66 | 0.65% | 0.1% |
| *15 | -- | -- | 0.55% | 0.11% | 0.55 | 0.65% | 0.1% |
| *16 | -- | -- | 0.55% | 0.11% | 0.44 | 0.65% | 0.1% |
| *17 | -- | -- | 0.55% | 0.11% | 0.33 | 0.65% | 0.1% |
| *18 | -- | -- | 0.55% | 0.11%. | 0.22 | 0.65% | 0.1% |
| *19 | -- | -- | 0.55% | 0.11% | 0.11 | 0.65% | 0.1% |
| *20 | -- | -- | 0.55% | 0.11% | 0.05 | 0.65% | 0.1% |

| | | | | | | | |
|---|---|---|---|---|---|---|---|
| NOTE: ⁽¹⁾Distilled Water up to 100.0. | | | | | | | |
| * = Comparative test | | | | | | | |

**TABLE IA**

| RESULTS | | | | | | | |
|---|---|---|---|---|---|---|---|
| Sol.# | ORGANISM LOG REDUCTION⁽¹⁾ | | | | | | Effective |
| | S. aureus | | P. aeruginosa | | E. coli | | |
| | T=14 | T=28 | T=14 | T=28 | T=14 | T=28 | |
| 1 | 6.5 | 5.4 | 6.5 | 5.4 | 6.2 | 5.0 | Pass |
| 2 | 6.5 | 5.4 | 6.5 | 5.4 | 6.2 | 5.0 | Pass |
| 3 | 6.5 | 5.4 | 6.5 | 5.4 | 6.2 | 5.0 | Pass |
| 4 | 6.5 | 5.4 | 6.5 | 5.4 | 6.2 | 5.0 | Pass |
| 5 | 6.5 | 5.4 | 6.5 | 5.4 | 6.2 | 5.0 | Pass |
| 6 | 6.5 | 5.4 | 6.5 | 5.4 | 6.2 | 5.0 | Pass |
| 7 | 6.5 | 5.4 | 6.5 | 5.4 | 6.2 | 5.0 | Pass |
| 8 | 6.5 | 5.4 | 6.5 | 5.4 | 4.7 | 5.0 | Pass |
| 9 | 6.5 | 5.4 | 6.5 | 5.4 | 4.4 | 5.0 | Pass |
| 10 | 6.5 | 5.4 | 6.5 | 5.4 | 4.3 | 5.0 | Pass |
| *11 | 6.5 | 5.4 | 1.5 | 1.9 | 6.2 | 5.0 | Fail |
| *12 | 6.5 | 5.4 | 1.1 | 1.3 | 4.2 | 3.7 | Fail |
| *13 | 6.5. | 5.4 | 0.6 | 0.7 | 3.6 | 1.4 | Fail |
| *14 | 6.5 | 5.4 | 0.6 | 0.3 | 3.3 | 1.6 | Fail |
| *15 | 6.5 | 5.4 | 0.4 | 0.1 | 1.7 | 1.5 | Fail |
| *16 | 6.5 | 5.4 | 0.3 | 0.3 | 1.0 | 1.2 | Fail |
| *17 | 6.5 | 5.4 | 0.3 | 0.3 | 0.4 | 0.7 | Fail |
| *18 | 6.5 | 5.4 | 0.1 | 0.3 | 1.0 | 1.6 | Fail |
| *19 | 6.5 | 5.4 | 0.2 | 0.4 | 1.6 | 3.2 | Fail |
| *20 | 6.5 | 5.4 | 0.1 | 0.5 | 1.5. | 3.3 | Fail |

| | | | | | | | |
|---|---|---|---|---|---|---|---|
| Note: ⁽¹⁾3 log reduction at 14 days and 28 days required for each test organisms. | | | | | | | |
| * = Comparative test | | | | | | | |

As shown by Tables I and IA borate buffered PHMB solutions evaluated are effective as preservative solutions at PHMB concentrations of 0.99 ppm or less whereas the phosphate buffered solutions are not effective at such PHMB concentrations.

### EXAMPLE VI

In this example the effectiveness of polyhexamethylene biguanide hydrochloride (n = 4.5 to 6.5) as a disinfecting agent is evaluated. Each disinfecting solution is prepared by the method of Example I. All ingredients are by weight percent unless otherwise noted. Following the microbial test methods of Example I, each solution is evaluated as a disinfectant against S. epidermidis, and C. albicans organisms after 5 hours. To be considered effective in this test, there must be at least 3 log reduction in the amount of S. epidermidis and no growth in C. albicans within 5 hours, the solutions and test results are tabulated in Table II below.

**TABLE II**

| PHMB DISINFECTING SOLUTIONS | | | | | | | | |
|---|---|---|---|---|---|---|---|---|
| FORMULATION⁽¹⁾ | | | | | | | ORGANISM LOG REDUCTION | |
| Sol. # | Sodium Chlor. | Boric Acid | Sodium Borate | Na₂ EDTA | Poloxamine 1107 | PHMB (ppm) | S. epidermidis T=5 Hrs.⁽²⁾ | C. albicans T=5 Hrs.⁽³⁾ |
| 1 | 0.49 | 0.64 | 0.16 | 0.11 | 0.5 | 0.88 | 6.0 | 1.3 |
| 2 | 0.29 | 1.0 | 0.29 | 0.11 | 0.5 | 0.88 | 6.0 | 1.4 |
| 3 | 0.49 | 0.64 | 0.16 | 0.11 | 1.0 | 0.88 | 6.0 | 1.5 |
| 4 | 0.29 | 1.0 | 0.29 | 0.11 | 1.0 | 0.88 | 6.0 | 1.9 |
| 5 | 0.49 | 0.64 | 0.16 | 0.11 | 0.5 | 0.99 | 6.0 | 1.5 |
| 6 | 0.29 | 1.0 | 0.29 | 0.11 | 0.5 | 0.99 | 6.0 | 1.7 |
| 7 | 0.49 | 0.64 | 0.16 | 0.11 | 1.0 | 0.99 | 6.0 | 1.5 |
| 8 | 0.29 | 1.0 | 0.29 | 0.11 | 1.0 | 0.99 | 4.6 | 2.0 |
| 9 | 0.29 | 1.0 | 0.29 | 0.11 | 0.5 | 0.55 | 4.7 | 0.4 |
| 10 | 0.49 | 0.64 | 0.16 | 0.11 | 1.0 | 0.55 | 4.2 | 0.4 |
| 11 | 0.49 | 0.64 | 0.16 | 0.11 | 1.0 | 0.55 | 3.7 | 1.9 |
| 12 | 0.49 | 0.64 | 0.12 | 0.055 | 1.0 | 0.55 | 4.4 | 2.4 |
| 13 | 0.49 | 0.64 | 0.12 | 0.022 | 1.0 | 0.55 | 4.0 | 2.9 |
| 14 | 0.49 | 0.68 | 0.12 | 0.01 | 1.0 | 0.55 | 4.3 | 4.2 |

| | | | | | | | | |
|---|---|---|---|---|---|---|---|---|
| Note: ⁽¹⁾ Distilled water qs to 100.0 | | | | | | | | |
| ⁽²⁾ 3 log reduction at 5 hours required. | | | | | | | | |
| ⁽³⁾ An indication of log reduction in 5 hours required. | | | | | | | | |

### EXAMPLE VII

The lens binding characteristics for a 0.005 percent aqueous solution of (PHMB) polyhexamethylene biguanide HCl (n = 4.5 to 6.5) is compared against an aqueous solution containing 0.005 percent chlorhexidine gluconate. The solutions are tested by the Zone of Inhibition Technique described in the Australian Journal of Optometry. Vol. 59, (1976) page 277 by Otten and Szabocsik.

Eight polymacon sort contact lenses available from Bausch & Lomb under the trademark SOFLENS® are exposed to the PHMB solution and another eight lenses exposed to the chlorhexidine solution. For a control an additional two lenses are placed in isotonic saline solution. All the lenses remain in their respective solutions overnight at room temperature. A lens from each of the solutions is rinsed with saline solution and placed on an agar plate seeded with microorganisms. Another two lenses from the PHMB and chlorhexidine solutions are soaked for 15 minutes in isotonic saline solution, rinsed in saline and placed on individual agar plates. Two more lenses previously soaked in PHMB and chlorhexidine are soaked in isotonic saline solution for one hour, rinsed in saline and placed on individual agar plates. The remaining lenses are soaked for three hours in isotonic saline, rinsed in saline and placed on individual agar plates. The two control lenses are rinsed in saline and placed on agar plates.

All the agar plates are incubated at elevated temperatures for 48 hours and the size (radius) of the zones of bacterial inhibition measured. The larger the zone of inhibition the greater the potential for preservative binding characteristics. The results are shown in Table III below.

**TABLE III**

| (BINDING PROPERTIES) | | | | |
|---|---|---|---|---|
| Preservative Solution | ZONE OF INHIBITION | | | |
| | Saline Rinse | Saline Soak . | | |
| | | 15 Min. | 1 Hour | 3 Hours |
| PHMB (50 ppm) | 1 mm | 0.5 mm | no zone | no zone |
| Chlorhexidine (50 ppm) | 7 mm | 8 mm | 7 mm | 5.5 mm |
| Control | no zone | --- | --- | --- |

Table III demonstrates the very low lens binding characteristics of PHMB.

### EXAMPLE VIII

This example demonstrates that PHMB does not cause a cytotoxic reaction when used as a disinfectant at 10 ppm concentration.

Comparative studies are conducted to evaluate the cytotoxicity of individual aqueous lens disinfecting and preservative solutions containing PHMB, benzalkonium chloride and chlorhexidine. The studies utilize the Agar Overlay Assay Technique published in the Journal of Pharmaceutical Sciences, Vol. 54 (1965) pp 1545-1547 by W. L. Guess et al. The technique utilized is similar to the Zone of Inhibition Method of Otten et al (Example VII), however, when the lenses are removed from the preservative solutions they are only rinsed with isotonic saline solution to remove residual preservative. The lenses are then placed on L-929 mouse fibroblast cells to ascertain the presence of any cytotoxic response or cell inflammation which is noted by a lysing of the cells. The results are shown in Table IV below.

**TABLE IV**

| (CYTOTOXICITY) | | |
|---|---|---|
| Preservative Solution | Cytotoxic Response | Width of Decolorized Zone Percent Cells Lysed |
| PHMB | | |
| (10 ppm)* | no | 0/0 |

| Chlorhexidine | | |
|---|---|---|
| ( 10 ppm)** | no | 0/0 |
| (100 ppm)* | yes | 2/5 |

| Benzalkonium Chloride | | |
|---|---|---|
| ( 10 ppm)** | no | 0/0 |
| (100 ppm)* | yes | 4/5 |
| 2 = zone not greater than 0.5 cm 4 = zone greater than 1.0 cm 5 = more than 80% of decolorized zone lysed | | |

| | | |
|---|---|---|
| * effective for disinfecting contact lens at this concentration. | | |
| ** not effective for disinfecting contact lens at this concentration. | | |

Table IV demonstrates that PHMB does not cause a cytotoxic reaction when used at a disinfecting concentration of 10 ppm. The solutions of chlorhexidine and benzalkonium chloride perform as positive controls. Thus, disinfecting/preserving solutions of this invention are non-cytotoxic.

### EXAMPLE IX

An aqueous contact lens disinfecting solution is prepared with the following formulation:

| | Percent |
|---|---|
| Polyhexamethylene Biguanide HCl* | .00011 |
| Poloxamine 1107 | .50 |
| Sodium Borate | .20 |
| Boric Acid | .60 |
| Sodium Chloride | .49 |
| Distilled Water qs | 100.0 |

| | |
|---|---|
| *n = 4.5 to 6.5 | |

The solution is prepared and sterilized following the procedures of Example I. Additional solutions are prepared in the same manner except the level of PHMB is 2 ppm, 3 ppm, 5 ppm, 10 ppm or 55 ppm.

The corneal staining property of each of the above solutions is evaluated in small patient clinical groups. The eyes of each patient are examined early in the morning to establish a baseline condition. Then the solution to be evaluated is administered to the patient's eyes. The eyes are observed immediately after application of the solution, fours later and eight hours later. The increase in corneal staining (redness) is noted for each patient. The limit for acceptable amount of increase in corneal staining is 11 percent. The results are tabulated in Table V below.

**TABLE V**

| Amount PHMB (ppm) | Percent Corneal Staining (Average for Group) |
|---|---|
| 1 | 7 |
| 2 | 5.5 |
| 3 | 9 |
| 5 | 17 |
| 10 | 21.5 |
| 55 | 62 |

| Controls: | |
|---|---|
| Commercial Product (with thimerosal) | |
| F | 35 |
| C* | 25 |

| | |
|---|---|
| *Todate, this commercial product has been considered the best commercial disinfecting solution. | |

Table IV, in combination with Table V, demonstrates that cytotoxicity and corneal staining are relatively independent functions. A contact lens solution may be non-cytotoxic and still not be acceptable to the patient. However, a contact lens solution which is cytotoxic will definitely be unacceptable to the patient as is the case of using 100 ppm chlorhexidine and BAK in contact lens solutions. On the table level of ocular irritation manifested in clinically observed ocular staining of 21.5 percent. The same explanation applies to 5 ppm PHMB, 10 ppm chlorhexidine and 10 ppm BAK.

## Claims

1. A disinfecting and/or preserving solution comprising (a) a microbicidally effective amount, within the range from 0.000001 to 0.0003 weight percent, of a biguanide, or a water-soluble salt thereof, having the formula: 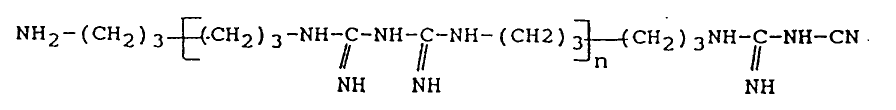 wherein n is from 1 to 500, and (b) a buffer, characterized in that said buffer is a borate buffer.

2. A solution according to Claim 1, wherein the biguanide is a polymeric water-soluble salt.

3. A solution according to Claim 2, wherein the biguanide is a water-soluble salt of polyhexamethylene biguanide.

4. A solution of Claim 2 or Claim 3, wherein the biguanide is a polymer having molecular weights less than 100,000.

5. A solution according to Claim 4, wherein the biguanide is a polymer having molecular weights in the range from 1,000 to 50,000.

6. A solution according to any one of Claims 3-5, wherein n of the biguanide polymer averages between 2 and 12.

7. A solution according to Claim 6, wherein n averages from 4 to 7.

8. A solution according to any preceding claim, wherein wherein the biguanide is present in an amount from about 0.00001 to about 0.0003 weight percent.

9. A solution according to any preceding claim, further including at least one member selected from a tonicity agent, a non-ionic surfactant, a cationic surfactant and a viscosity builder.

10. A solution according to any preceding claim, further including at least one other germicidal agent compatible with said biguanide.

11. A method of disinfecting and/or preserving contact lenses, which comprises contacting said lenses with a germicidally effective amount of a solution according to any preceding claim.

12. The method of Claim 11, including the step of rinsing the lenses of residual disinfecting solution.

## Patentansprüche

1. Desinfektions- und/oder Aufbewahrungslösung, die (a) eine mikrobizid wirkende, im Bereich von 0,000001 bis 0,0003 Gew.-%, liegende Menge eines Diguanids oder eines wasserlöslichen Salzes davon mit der Formel 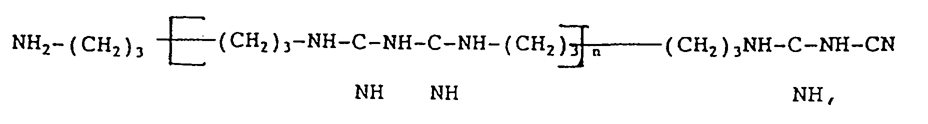 wobei n 1 bis 500 ist, und (b) einen Puffer umfaßt, dadurch gekennzeichnet, daß der Puffer ein Boratpuffer ist.

2. Lösung nach Anspruch 1, bei der das Diguanid ein polymeres, wasserlösliches Salz ist.

3. Lösung nach Anspruch 2, bei der das Diguanid ein wasserlösliches Polyhexamethylendiguanidsalz ist.

4. Lösung nach Anspruch 2 oder Anspruch 3, bei der das Diguanid ein Polymer mit einem Molekulargewicht kleiner als 100 000 ist.

5. Lösung nach Anspruch 4, bei der das Diguanid ein Polymer mit einem Molekulargewicht im Bereich von 1000 bis 50 000 ist.

6. Lösung nach einem der Ansprüche 3 bis 5, bei der n im Diguanidpolymer durchschnittlich 2 bis 12 beträgt.

7. Lösung nach Anspruch 6, bei der n durchschnittlich 4 bis 7 beträgt.

8. Lösung nach einem der vorhergehenden Ansprüche, bei der das Diguanid in einer Menge von etwa 0,00001 bis etwa 0,0003 Gew.-% vorhanden ist.

9. Lösung nach einem der vorhergehenden Ansprüche, die außerdem mindestens einen Bestandteil ausgewählt aus einem Tonizitätsmittel, einem nicht-ionischen Tensid, einem kationischen Tensid und einem Viskositätserzeuger enthält.

10. Lösung nach einem der vorhergehenden Ansprüche, die außerdem mindestens ein anderes keimtötendes Mittel enthält, das mit dem Diguanid kompatibel ist.

11. Verfahren zur Desinfektion und/oder Aufbewahrung von Kontaktlinsen, bei dem die Linsen mit einer keimtötend wirkenden Menge einer Lösung gemäß einem der vorhergehenden Ansprüche kontaktiert werden.

12. Verfahren nach Anspruch 11, bei dem zurückbleibende Desinfektionslösung von den Linsen abgespült wird.

## Revendications

1. Solution de désinfection et/ou de conservation comprenant (a) une quantité microbicidement efficace de l'ordre de 0,000001 à 0,0003 % en poids d'un biguanide, ou un sel soluble de ce dernier, possédant la formule: 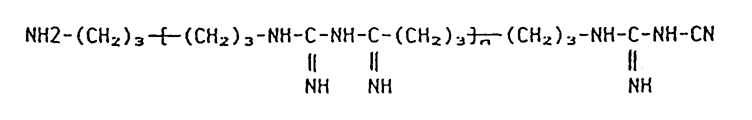 dans laquelle n va de 1 à 500, et (b) est un tampon, caractérisé en ce que ledit tampon est un tampon de borate.

2. Solution selon la revendication 1, dans laquelle le biguanide est un sel polymère soluble dans l'eau.

3. Solution selon la revendication 2, dans laquelle le biguanide est un sel soluble dans l'eau de polyhexaméthylène biguanide.

4. Solution selon la revendication 2 ou la revendication 3, dans laquelle le biguanide est un polymère dont la masse moléculaire est intérieure à 100 000.

5. Solution selon la revendication 4, dans laquelle le biguanide est un polymère dont la masse moléculaire se situe entre 1 000 et 50 000.

6. Solution selon l'une quelconque des revendications 3 à 5, dans laquelle le coefficient n du polymère de biguanide se situe en moyenne entre 2 et 12.

7. Solution selon la revendication 6, dans laquelle n se situe en moyenne entre 4 et 7.

8. Solution selon l'une quelconque des revendications précédentes, dans laquelle le biguanide est présent à raison d'une quantité comprise entre 0,00001 et 0,0003 en poids environ.

9. Solution selon l'une quelconque des revendications précédentes, comprenant en outre au moins un élément choisi parmi un agent de tension, un agent tensio-actif non ionique, un agent tensio-actif cationique, et un adjuvant de viscosité.

10. Solution selon l'une quelconque des revendications précédentes, comportant en outre au moins un autre agent germicide compatible avec ledit biguanide.

11. Procédé de désinfection et/ou de conservation de lentilles de contact, qui comprend la mise en contact de ces lentilles avec une quantité efficace du point de vue germicide d'une solution selon l'une quelconque des revendications précédentes.

12. Procédé selon la revendication 11, comprenant l'étape de rinçage des lentilles des restes de la solution désinfectante.
